(19) Europäisches Patentamt

European Patent Office

Office européen des brevets

(11) **EP 4 300 022 A1**

(12) **EUROPEAN PATENT APPLICATION**
published in accordance with Art. 153(4) EPC

(43) Date of publication:
**03.01.2024 Bulletin 2024/01**

(21) Application number: **22775720.0**

(22) Date of filing: **23.03.2022**

(51) International Patent Classification (IPC):
*F26B 25/06* (2006.01)    *A23L 3/44* (2006.01)
*C07K 14/47* (2006.01)    *C12M 1/00* (2006.01)
*F26B 5/06* (2006.01)

(52) Cooperative Patent Classification (CPC):
**A23L 3/44; C07K 14/47; C12M 1/00; F26B 5/06; F26B 25/06**

(86) International application number:
**PCT/JP2022/013715**

(87) International publication number:
**WO 2022/202938 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2021 JP 2021054136**

(71) Applicant: **FUJIFILM Corporation**
**Tokyo 106-8620 (JP)**

(72) Inventors:
• **MOCHIZUKI, Yusuke**
**Ashigarakami-gun,**
**Kanagawa 258-8577 (JP)**

• **FUSHIMI, Hideo**
**Ashigarakami-gun,**
**Kanagawa 258-8577 (JP)**
• **KATO, Nobuhiko**
**Ashigarakami-gun,**
**Kanagawa 258-8577 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **FREEZE-DRYING CONTAINER**

(57)    The present disclosure provides a freeze-drying container which includes a container body storing liquid to be freeze-dried and a first heat transfer body being in contact with the liquid to be freeze-dried and a cold source and in which the container body is a non-heat transfer body or a second heat transfer body of which at least a part is in contact with a liquid surface of the liquid to be freeze-dried and the cold source and at least one of a first shortest distance between an arbitrary point on the liquid surface of the liquid to be freeze-dried and the first heat transfer body or a second shortest distance in the liquid surface between the arbitrary point on the liquid surface of the liquid to be freeze-dried and the second heat transfer body is 20 mm or less.

FIG. 1

**EP 4 300 022 A1**

**Description**

**BACKGROUND OF THE INVENTION**

1. Field of the Invention

The present disclosure relates to a freeze-drying container.

2. Description of the Related Art

A freeze-drying container is widely used to freeze-dry various types of liquid to be freeze-dried. Such a freeze-drying container is disclosed in, for example, JP1987-101728U (JP-S62-101728U).

**SUMMARY OF THE INVENTION**

A technique for obtaining a freeze-dried body including uniform pores is required from the viewpoint of improving a quality.
The present disclosure has been made in consideration of the above-mentioned circumstances, and an object to be achieved by an embodiment of the present disclosure is to provide a freeze-drying container from which a freeze-dried body including uniform pores can be obtained.
The present disclosure includes the following aspects.

<1> A freeze-drying container comprising:

a container body that stores liquid to be freeze-dried; and
a first heat transfer body that is in contact with the liquid to be freeze-dried and a cold source,
in which the container body is a non-heat transfer body or a second heat transfer body of which at least a part is in contact with a liquid surface of the liquid to be freeze-dried and the cold source, and
at least one of a first shortest distance between an arbitrary point on the liquid surface of the liquid to be freeze-dried and the first heat transfer body or a second shortest distance in the liquid surface between the arbitrary point and the second heat transfer body is 20 mm or less.

<2> The freeze-drying container according to <1>,
in which a ratio of an area of portions of the first heat transfer body and the second heat transfer body, which are in contact with the liquid to be freeze-dried, to a volume of the liquid to be freeze-dried stored in the freeze-drying container at a temperature of 23°C is 200 mm$^2$/mL or more.
<3> The freeze-drying container according to <1> or <2>,
in which a thermal conductivity of the first heat transfer body at a temperature of 0°C is 15 W/m·K or more.
<4> The freeze-drying container according to any one of <1> to <3>,
in which a thermal conductivity of the second heat transfer body at a temperature of 0°C is 15 W/m·K or more.
<5> The freeze-drying container according to any one of <1> to <4>,
in which the first heat transfer body is disposed in a direction perpendicular to a bottom surface of the container body.
<6> The freeze-drying container according to any one of <1> to <5>,
in which at least a part of portions of the container body and the first heat transfer body, which are in contact with the liquid to be freeze-dried, include a hydrophobic film.
<7> The freeze-drying container according to <6>,
in which an entirety of the container body and the first heat transfer body includes the hydrophobic film.
<8> The freeze-drying container according to <6> or <7>,
in which a thickness of the hydrophobic film is 1 mm or less.

According to an embodiment of the present disclosure, there is provided a freeze-drying container from which a freeze-dried body including uniform pores can be obtained.

**BRIEF DESCRIPTION OF THE DRAWINGS**

Fig. 1 is a schematic perspective view showing an example of a freeze-drying container.

Fig. 2 is a schematic plan view showing the example of the freeze-drying container.

Fig. 3 is a schematic plan view showing an example of the freeze-drying container.

Fig. 4 is a schematic perspective view showing an example of the freeze-drying container.

Fig. 5 is a schematic plan view showing the example of the freeze-drying container.

Fig. 6 is a schematic cross-sectional view showing an example of the freeze-drying container.

Fig. 7 is a schematic cross-sectional view showing an example of an aspect in which liquid to be freeze-dried is stored in the freeze-drying container.

Fig. 8 is a schematic cross-sectional view showing an example of an aspect in which liquid to be freeze-dried is stored in the freeze-drying container.

Fig. 9 is an optical microscope image of a tissue section of a freeze-dried body of Example 1.

Fig. 10 is an optical microscope image of a specimen of a freeze-dried body of Example 2.

Fig. 11 is an optical microscope image of a specimen of a freeze-dried body of Example 3.

Fig. 12 is an optical microscope image of a specimen of a freeze-dried body of Example 4.

Fig. 13 is an optical microscope image of a specimen of a freeze-dried body of Example 5.

Fig. 14 is an optical microscope image of a specimen of a freeze-dried body of Example 6.

Fig. 15 is an optical microscope image of a specimen of a freeze-dried body of Comparative Example 1.

## DESCRIPTION OF THE PREFERRED EMBODIMENTS

Details of a freeze-drying container according to an embodiment of the present disclosure will be described.

A numerical range described using "to" in the present disclosure means a range that includes numerical values written in the front and rear of "to" as the minimum value and the maximum value, respectively.

Regarding numerical ranges that are described stepwise in the present disclosure, an upper limit or a lower limit described in a certain numerical range may be replaced with an upper limit or a lower limit of another stepwise numerical range. Further, in the numerical ranges described in the present disclosure, an upper limit or a lower limit disclosed in a certain range may be replaced with values shown in Examples.

In the present disclosure, a combination of two or more preferred aspects is a more preferred aspect.

In the present disclosure, in a case where plural kinds of substances corresponding to each component are present, an amount of each component means the total amount of the plural kinds of substances, unless otherwise noted.

In the present disclosure, the term "step" includes not only an independent step but also a step of which an intended purpose is achieved even in a case where the step cannot be clearly distinguished from other steps.

In the present disclosure, an amino acid sequence of a polypeptide may be expressed in one-letter notation (for example, "G" in the case of a glycine residue) or three-letter notation (for example, "Gly" in the case of a glycine residue) that is well known in the art.

In the present disclosure, "%" related to an amino acid sequence of a polypeptide is based on the number of amino acid (or iminoic acid) residues unless otherwise specified. In the present disclosure, "identity" related to amino acid sequences of two kinds of polypeptides to be contrasted with each other refers to a value calculated from the following equation.

The contrast (alignment) of the plurality of polypeptides is performed according to a usual method such that the number of identical amino acid residues is the largest.

$$\text{Identity (\%)} = \{(\text{number of identical amino acid residues}/(\text{alignment length})\} \times 100$$

The drawings referred to in the following description are exemplarily and schematically illustrated, and the present disclosure is not limited to the drawings. The same reference numerals denote the same components. Further, reference numerals in the drawings may be omitted.

<Freeze-drying container>

A freeze-drying container according to an embodiment of the present disclosure includes a container body that stores liquid to be freeze-dried, and a first heat transfer body that is in contact with the liquid to be freeze-dried and a cold source.

The container body is a non-heat transfer body or a second heat transfer body of which at least a part is in contact with a liquid surface of the liquid to be freeze-dried and the cold source.

Further, at least one of a first shortest distance between an arbitrary point on the liquid surface of the liquid to be freeze-dried and the first heat transfer body or a second shortest distance in the liquid surface between the arbitrary point and the second heat transfer body is 20 mm or less.

A freeze-dried body, which is obtained in a case where the liquid to be freeze-dried is freeze-dried, has a pore structure, and pores are included in the freeze-dried body as air bubbles. A freeze-drying container comprising a partition plate disclosed in JP1987-101728U (JP-S62-101728U) is known as a freeze-drying container. However, even though the freeze-drying container comprising a partition plate is used simply, the sizes and present state of pores formed in a dried body after the freeze-dried body are likely to be non-uniform. For this reason, it may be difficult to obtain a freeze-dried body that includes uniform pores.

On the other hand, in the freeze-drying container according to the embodiment of the present disclosure, at least one of the first shortest distance between an arbitrary point on a liquid surface of the liquid to be freeze-dried and the first heat transfer body or the second shortest distance in the liquid surface between the arbitrary point and the second heat transfer body is 20 mm or less. That is, since the arbitrary point on the liquid surface of the liquid to be freeze-dried can be in a range of 20 mm or less from at least one of the first heat transfer body or the second heat transfer body, a freeze-dried body including uniform pores can be obtained.

The first heat transfer body and the second heat transfer body may be simply referred to as "heat transfer bodies".

In the present disclosure, the "heat transfer body" refers to a material having a thermal conductivity of 5 W/m•K or more at a temperature of 0°C. The thermal conductivity is a value that is measured by a laser flash method. A material other than a heat transfer body is referred to as a "non-heat transfer body".

[First heat transfer body]

The shape of the first heat transfer body is not particularly limited, and may be the shape of, for example, a plate, a rod, or the like.

A position of the first heat transfer body disposed in the container body is not particularly limited. For example, the first heat transfer body, which is in contact with the liquid to be freeze-dried and the cold source, may be disposed in a direction perpendicular to a bottom surface of the container body. Accordingly, in a case where the cold source is disposed in contact with a bottom portion of the container body, the first heat transfer body can be efficiently cooled via the bottom portion. Here, "perpendicular" means that an angle is 90°±15°.

A material of the first heat transfer body is not particularly limited as long as the thermal conductivity of the first heat transfer body is 5 W/m•K or more at a temperature of 0°C. It is preferable that the material of the first heat transfer body is, for example, metal. The metal is preferably, for example, aluminum, gold, silver, copper, iron, an alloy thereof (for example, an aluminum alloy (for example, A5052), stainless steel, or the like), and more preferably aluminum or an alloy thereof.

From the viewpoint of obtaining a freeze-dried body including uniform pores in a short time, the thermal conductivity of the first heat transfer body at a temperature of 0°C is preferably 15 W/m•K or more, more preferably 50 W/m•K or more, still more preferably 100 W/m•K or more, and even more preferably 150 W/m•K or more.

One first heat transfer body may be provided or two or more first heat transfer bodies may be provided. In a case where a plurality of first heat transfer bodies are provided, the first heat transfer bodies may be independent of each other or at least a part of these first heat transfer bodies may be connected to each other.

In a case where a plurality of first heat transfer bodies are provided and at least a part of these first heat transfer bodies are connected to each other, an aspect of the connection is not particularly limited. It is preferable that the first heat transfer bodies are thermally connected to each other, and thermal conductivity between the first heat transfer bodies connected to each other is preferably 15 W/m•K or more, more preferably 50 W/m•K or more, and still more preferably 100 W/m•K or more.

Further, at least a part of the first heat transfer body and the second heat transfer body may be connected to each other. An aspect of the connection is not particularly limited, and it is preferable that at least a part of the first heat transfer body and the second heat transfer body are thermally connected to each other. Thermal conductivity between the first heat transfer body and the second heat transfer body connected to each other is preferably 15 W/m•K or more, more preferably 50 W/m•K or more, and still more preferably 100 W/m•K or more.

The first heat transfer body may function as a partition member. Accordingly, the freeze-drying container may be provided with a plurality of storage portions that store the liquid to be freeze-dried and do not communicate with each other. The plurality of storage portions may store the same liquid to be freeze-dried and may store different types of liquid to be freeze-dried. The plurality of storage portions may store the same volume of the liquid to be freeze-dried, or may store different volumes of the liquid to be freeze-dried.

[Container]

The container body is a container that stores the liquid to be freeze-dried. The container body is a non-heat transfer body or a second heat transfer body of which at least a part is in contact with a liquid surface of the liquid to be freeze-dried and the cold source.

The shape of the container body is not particularly limited, and may be, for example, a rectangular parallelepiped shape, a tubular shape, or the like.

A material of the container body is not particularly limited, and examples of the material of the container body include a non-heat transfer body, such as a resin, a second heat transfer body, such as metal, and the like.

The size of the container body is not particularly limited, and a freeze-dried body including uniform pores can be obtained even in a case where the size of the container body is large.

On the other hand, in the case of a freeze-drying container in which, for example, a container body is formed of a heat transfer body but a heat transfer body is not disposed in the container body (hereinafter, referred to as a "container in the related art"), it is necessary to make the container body small to perform uniform freeze-drying. That is, a storage portion of the container body of the container in the related art is narrow in a plan view, so that a distance between an arbitrary point on the liquid to be freeze-dried and the container body (heat transfer body) is short. Accordingly, it is easy to perform uniform freeze-drying. That is, in the case of the container in the related art, the maximum length of a contour of the storage portion of the container body (that is, the maximum value of the length of a straight line connecting two arbitrary points on the contour) is less than about 20 mm in a plan view. The maximum length of the contour is, for example, the length of a diagonal line in a case where the storage portion of the container body has a quadrangular shape, is a diameter in a case where the storage portion has a circular shape, and is the length of a major axis in a case where the storage portion has an elliptical shape.

Even though the storage portion of the container body of the freeze-drying container according to the embodiment of the present disclosure is wide in a plan view, uniform freeze-drying can be performed and a freeze-dried body including uniform pores can be obtained Since a heat transfer body is disposed in the container body and a shortest distance between an arbitrary point on a liquid surface of the liquid to be freeze-dried and the heat transfer body is set to 20 mm or less. The maximum length of the contour of the storage portion of the container body may be, for example, 30 mm or more, 50 mm or more, or 90 mm or more in a plan view.

Further, the freeze-drying container according to the embodiment of the present disclosure is effective in freeze-drying a large amount of liquid to be freeze-dried.

For example, in a case where a large amount of liquid to be freeze-dried is to be freeze-dried in the case of the above-mentioned container in the related art, the container requires a certain length (for example, 5 cm) in a depth direction of the container to ensure a volume. Since the volume of the liquid to be freeze-dried is increased in the case of such a specific container, a difference in a dry state of the liquid to be freeze-dried is likely to occur between an upper portion and a lower portion of the specific container and it may be difficult to obtain a freeze-dried body including uniform pores.

On the other hand, in the freeze-drying container according to the embodiment of the present disclosure, the size of the container body can be appropriately selected and the heat transfer body can be appropriately provided according to the amount of liquid to be freeze-dried to be subjected to freeze-drying. Accordingly, even in a case where a large amount of liquid to be freeze-dried is to be freeze-dried, the volume of the liquid to be freeze-dried can be set in an appropriate range and it is easy to obtain a freeze-dried body including uniform pores.

In a certain aspect, the entire container body may be one second heat transfer body. In such an aspect, the freeze-drying container may be, for example, a container in which one or more first heat transfer bodies are connected to the inside of the container body consisting of the second heat transfer body.

In another aspect, the entire freeze-drying container may be one (that is, integrated) heat transfer body. In such an aspect, the freeze-drying container may be, for example, a container in which a first heat transfer body integrated with a container body consisting of a second heat transfer body is disposed in the container body. For example, metal can be cut to produce the freeze-drying container of such an aspect such that the second heat transfer body, which is the container body, and the first heat transfer body are integrated with each other.

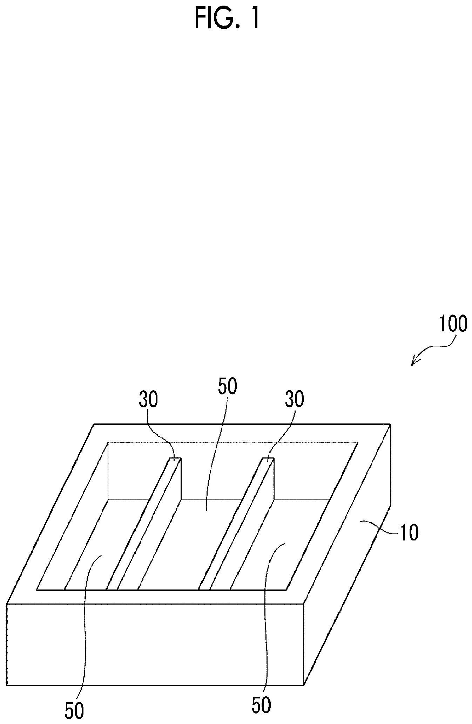

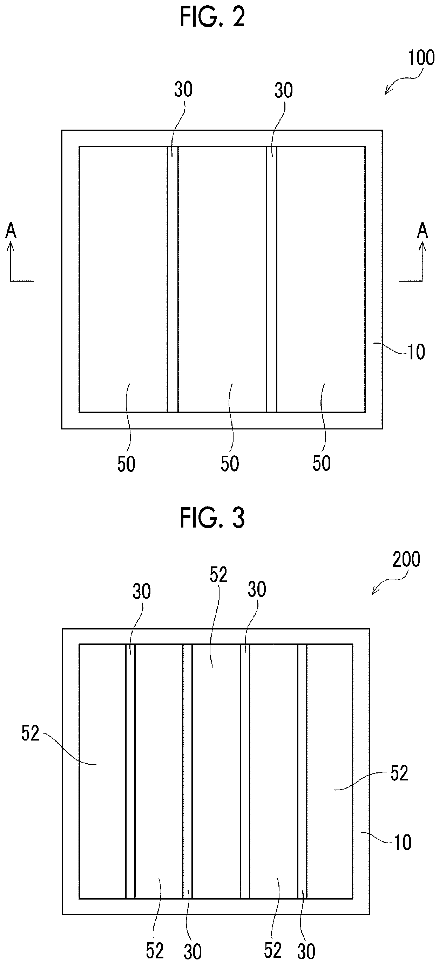

For example, a freeze-drying container 100 shown in Figs. 1 and 2 includes a container body 10 that has a rectangular parallelepiped shape and two plate-like first heat transfer bodies 30 that are perpendicular to a bottom surface of the container body 10, and the first heat transfer bodies 30 function as partition plates and partition the freeze-drying container into three storage portions 50 storing liquid to be freeze-dried.

For example, a freeze-drying container 200 shown in Fig. 3 includes a container body 10 that has a rectangular parallelepiped shape and four plate-like first heat transfer bodies 30 that are perpendicular to a bottom surface of the container body 10, and the first heat transfer bodies 30 function as partition plates and partition the freeze-drying container into five storage portions 50 storing liquid to be freeze-dried.

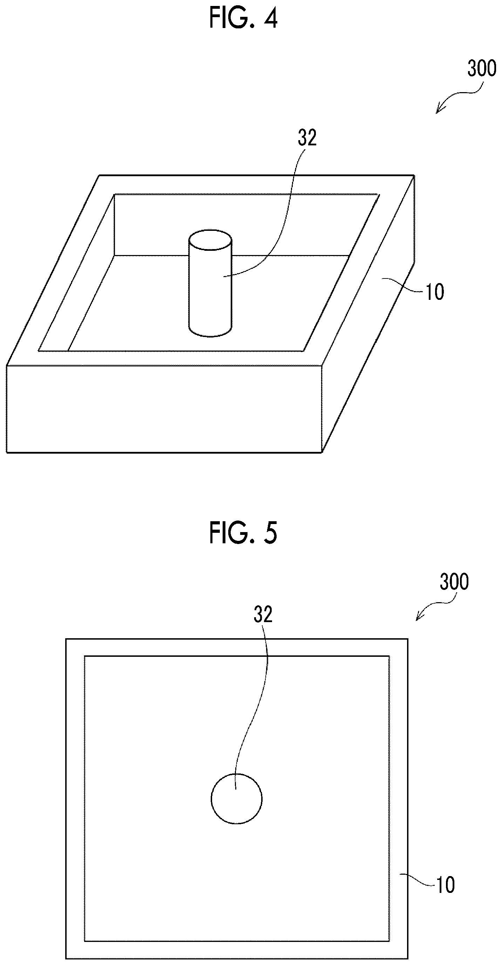

For example, a freeze-drying container 300 shown in Figs. 4 and 5 includes a container body 10 that has a rectangular parallelepiped shape and a rod-like first heat transfer body 32 that is perpendicular to a bottom surface of the container body 10.

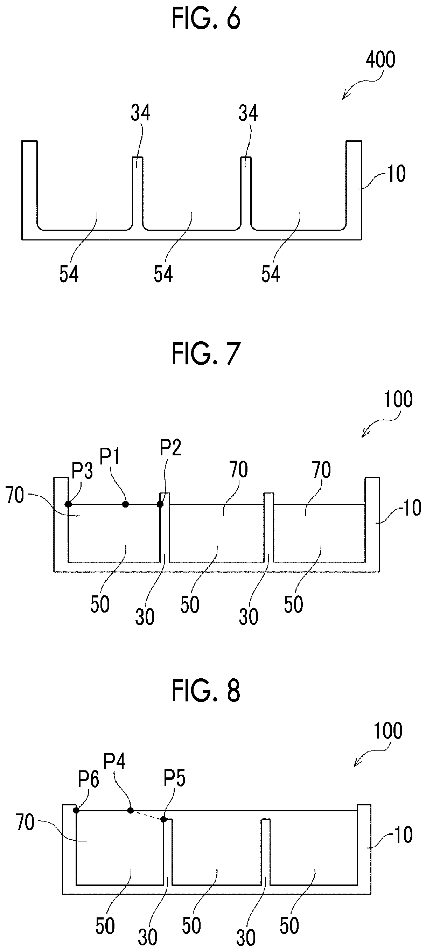

For example, as shown in Fig. 6, the shapes of longitudinal cross sections of first heat transfer bodies 34 connected to a container body 10 may be shapes rounded with respect to a bottom surface so that freeze-dried bodies are easily taken out of a freeze-drying container. Further, the shape of the longitudinal cross section may be a tapered shape, which is inclined, in addition to a round shape.

In the freeze-drying container, at least one of a first shortest distance between an arbitrary point on a liquid surface of the liquid to be freeze-dried (hereinafter, simply referred to as a "first shortest distance") or a second shortest distance in the liquid surface between the arbitrary point and a second heat transfer body (hereinafter, simply referred to as a "second shortest distance") is 20 mm or less. In a case where a container body is a non-heat transfer body, the first shortest distance is 20 mm or less. Accordingly, a freeze-dried body including uniform pores can be obtained.

For example, with regard to the freeze-drying container 100 shown in Figs. 1 and 2, in a case where liquid 70 to be freeze-dried is stored up to a position lower than the height of the first heat transfer body 30 as shown in Fig. 7, for example, a first shortest distance between an arbitrary point P1 on a liquid surface and a point P2 on the first heat transfer body 30 is 20 mm or less.

Further, in a case where at least a part of the container body 10 is the second heat transfer body, for example, at least one of the first shortest distance between the points P1 and P2 or a second shortest distance in the liquid surface between the point P1 and a point P3 on the second heat transfer body (a part of the container body 10) is 20 mm or less.

For example, with regard to the freeze-drying container 100 shown in Figs. 1 and 2, in a case where the liquid 70 to be freeze-dried is stored up to a position higher than the height of the first heat transfer body 30 as shown in Fig. 8, for example, a first shortest distance between an arbitrary point P4 on the liquid surface and a point P5 on the first heat transfer body 30 in the liquid 70 to be freeze-dried is 20 mm or less.

Further, in a case where at least a part of the container body 10 is the second heat transfer body, for example, at least one of the first shortest distance between the points P4 and P5 or a second shortest distance in the liquid surface between the point P4 and a point P6 on the second heat transfer body (a part of the container body 10) is 20 mm or less.

From the viewpoint of obtaining a freeze-dried body including uniform pores in a short time, it is preferable that the first shortest distance is 15 mm or less. Further, from the viewpoint of making pores more uniform, it is preferable that the first shortest distance is 5 mm or more.

Likewise, from the viewpoint of obtaining a freeze-dried body including uniform pores in a short time, it is preferable that the second shortest distance is 15 mm or less. Further, from the viewpoint of making pores more uniform, it is preferable that the second shortest distance is 5 mm or more.

A material of the second heat transfer body is not particularly limited as long as the thermal conductivity of the second heat transfer body is 5 W/m•K or more at a temperature of 0°C. It is preferable that the material of the second heat transfer body is, for example, metal. The metal is preferably, for example, aluminum, gold, silver, copper, iron, an alloy thereof (for example, an aluminum alloy (for example, A5052), stainless steel, or the like), and more preferably aluminum or an alloy thereof.

From the viewpoint of obtaining a freeze-dried body including uniform pores in a short time, the thermal conductivity of the second heat transfer body at a temperature of 0°C is preferably 15 W/m•K or more, more preferably 50 W/m•K or more, still more preferably 100 W/m•K or more, and even more preferably 150 W/m•K or more.

From the viewpoint of the shape stability of the container, a coefficient of thermal expansion of the material of the container body is preferably $10 \times 10^{-5}$/K or less, more preferably $50 \times 10^{-6}$/K or less, and still more preferably $25 \times 10^{-6}$/K or less. From such a viewpoint, it is preferable that the material of the container body is, for example, metal.

It is preferable that a ratio of an area of portions of the first heat transfer body and the second heat transfer body (the first heat transfer body in a case where the container body does not include the second heat transfer body), which are in contact with the liquid to be freeze-dried, to the volume of the liquid to be freeze-dried stored in the freeze-drying container at a temperature of 23°C (hereinafter, referred to as a "ratio of a liquid contact area to a liquid volume") is 200 mm$^2$/mL or more. Accordingly, a contact area between the liquid to be freeze-dried and the heat transfer bodies per unit volume can be increased. For this reason, it is easier to obtain a freeze-dried body, which includes uniform pores, in a short time. It is more preferable that the ratio of a liquid contact area to a liquid volume is 210 mm$^2$/mL or more.

The ratio of a liquid contact area to a liquid volume is preferably 1500 mm$^2$/mL or less, more preferably 1000 mm$^2$/mL or less, and still more preferably 500 mm$^2$/mL or less. Accordingly, since a contact area between the liquid to be freeze-dried and the first heat transfer body and the second heat transfer body (the first heat transfer body in a case where the container body does not include the second heat transfer body) per unit volume can be increased, the volume of the liquid to be freeze-dried per unit contact area of the heat transfer bodies can be increased, that is, the height (volume) of the liquid to be freeze-dried can be increased. In a case where freeze-dried bodies are taken out of the freeze-drying container, the detachability of the freeze-dried bodies from the freeze-drying container is further improved.

In a case where the freeze-drying container is partitioned into a plurality of portions as described above, "the ratio of a liquid contact area to a liquid volume satisfies the above-mentioned range" means that the ratio of a liquid contact area to a liquid volume satisfies the above-mentioned range in at least one storage portion.

The shapes, sizes, and the like of the container body, the first heat transfer body, and the second heat transfer body may be appropriately selected in consideration of the volume and the like of the liquid to be freeze-dried stored in the freeze-drying container such that the ratio of a liquid contact area to a liquid volume is in the above-mentioned range.

In the freeze-drying container, a hydrophobic film may be provided on at least a part of portions of the container body (which may include the second heat transfer body) and the first heat transfer body, which are in contact with the liquid to be freeze-dried, or a hydrophobic film may be provided on the entirety of the container body (which may include the second heat transfer body) and the first heat transfer body so that freeze-dried bodies are more easily taken out of the freeze-drying container. Accordingly, in a case where the freeze-drying container is taken out of freeze-dried bodies, the detachability of the freeze-dried bodies from the freeze-drying container is further improved.

A material of the hydrophobic film is not particularly limited, and examples of the material of the hydrophobic film include a fluorine-based material, a fluorine-based resin, a silicone resin, an aromatic polymer, a polyethylene-based resin, a polypropylene-based resin, a polystyrene-based resin, and the like. It is preferable that the hydrophobic film contains a fluorine-based material or an aromatic polymer, and it is particularly preferable that the hydrophobic film contains a fluorine-based material.

Examples of the fluorine-based material include a tetrafluoroethylene-hexafluoropropylene copolymer (FEP) (also referred to as a perfluoroethylene propene copolymer), a eutectic material of a fluororesin and nickel, and the like.

Hydrophobic treatment may be performed on a desired portion of the freeze-drying container to form the hydrophobic film. A method of performing hydrophobic treatment is not particularly limited, and examples of the method of performing hydrophobic treatment include solution coating, plating, vapor deposition, and the like. Hydrophobic plating may be performed on the freeze-drying container or components thereof by a publicly known method. Further, examples of the method of performing hydrophobic treatment include a method of forming a eutectic coating of a fluororesin and nickel using "NIFGRIP" (registered trademark) manufactured by ULVAC, Inc. or "KANIFLON" (registered trademark) manufactured by Japan Kanigen Co., Ltd. Furthermore, examples of the method of performing hydrophobic treatment include a method of forming an aromatic polymer film using the vapor deposition of parylene manufactured by Specialty Coating Systems, Inc.

A thickness of the hydrophobic film is not particularly limited. From the viewpoint of thermal conductivity between the heat transfer body and the liquid to be freeze-dried, the thickness of the hydrophobic film is preferably 1 mm or less, more preferably 500 $\mu$m or less and more preferably 250 $\mu$m or less. A lower limit of the thickness of the hydrophobic film may be about 0.5 $\mu$m.

The freeze-drying container may include members other than the above-mentioned members. The freeze-drying container may include, for example, a partition member other than the heat transfer body.

<Cold source>

The cold source is in contact with the heat transfer body that is in contact with the liquid to be freeze-dried, and freezes the liquid to be freeze-dried via the heat transfer body.

The cold source is not particularly limited as long as the liquid to be freeze-dried can be frozen, and may include, for example, a solid, a liquid, a supercritical fluid, or the like having a temperature of 0°C or less as a refrigerant. An aspect of the cold source is not particularly limited, and it is preferable that the cold source is a metal plate in which a refrigerant as a liquid flows.

The number of cold sources, which are in contact with the freeze-drying container, is not particularly limited, and one cold source or two or more cold sources may be provided. The number of cold sources, which are in contact with the freeze-drying container, may be appropriately selected in consideration of, for example, the number of the first heat transfer bodies, the presence or absence of the second heat transfer body, the sizes of the heat transfer bodies, the presence or absence of thermal connection between the heat transfer bodies, and the like. For example, in the case of a freeze-drying container of which a container body is a second heat transfer body and in which a first heat transfer body and the second heat transfer body are integrated with each other, the first heat transfer body and the second heat transfer body are thermally connected to each other as a whole. Accordingly, one cold source may be disposed on the bottom surface of the freeze-drying container to perform freeze-drying.

<Liquid to be freeze-dried>

The liquid to be freeze-dried is not particularly limited, and examples of the liquid to be freeze-dried include a biocompatible polymer, such as gelatin or collagen, an aqueous solution of the polymer, and the like.

Hereinafter, a case where a polymer aqueous solution of a biocompatible polymer, such as gelatin or collagen, (liquid to be freeze-dried) is freeze-dried to manufacture a polymer porous body (freeze-dried body) will be used as an example to further describe a method of manufacturing a freeze-dried body using the freeze-drying container according to the embodiment of the present disclosure.

-Polymer porous body-

It is preferable that the polymer porous body (freeze-dried body) is a porous block made of a polymer.

As described later, after a polymer aqueous solution (liquid to be freeze-dried) containing a polymer is put in the freeze-drying container and a freezing step is performed to obtain a polymer aqueous solution-frozen body in which the polymer aqueous solution is frozen, a water removal step is performed on the polymer aqueous solution-frozen body to remove water. As a result, the polymer porous body is obtained.

The polymer porous body may be subjected to a pulverization step, a classification step, a crosslinking step, and the like as needed, and is suitable as materials, such as cell scaffolds, a member for transplantation, and a tissue repair material.

-Polymer-

In the present disclosure, the term "polymer" refers to a molecule having a large molecular weight and having a structure in which a unit obtained substantially or conceptually from a molecule having a small molecular weight is repeated many times. Examples of the polymer include polyamine, cellulose, amylose, starch, chitin, polypeptide, protein, DNA, RNA, and the like. The polymer is preferably water-soluble, and is more preferably a polypeptide or protein. Collagen and gelatin are particularly preferable among polypeptides and proteins.

A ratio of hydrophilic repeating units in the polymer is preferably 50% or less and more preferably 30% or less. In a case where a ratio of hydrophilic units is higher than this ratio, the amount of free water around the polymer is reduced and freezing is inhibited. Here, the ratio of hydrophilic repeating units refers to a ratio of repeating units including an ionic group and/or a hydroxyl group in the polymer.

The gelatin means a polypeptide containing 6 or more consecutive sequences each of which is represented by Gly-X-Y, and may contain one or more other amino acid residues in the polypeptide other than the sequence represented by Gly-X-Y The sequence represented by Gly-X-Y is a sequence corresponding to an amino acid sequence derived from a partial amino acid sequence of collagen, and the repetition of this sequence means a sequence characteristic of collagen.

The plurality of Gly-X-Y sequences may be the same or different from each other. Further, X and Y in the Gly-X-Y sequence are independent for each repeating unit, and may be the same or different from each other. In Gly-X-Y, Gly represents a glycine residue and X and Y represent arbitrary amino acid residues other than the glycine residue. It is preferable that X and Y contain a large amount of iminoic acid residues, that is, proline residues or oxyproline residues. It is preferable that the content of such imino acid residue is in a range of 10% to 45% of the entire gelatin. The content of Gly-X-Y in the gelatin is preferably 80% or more, more preferably 95% or more, and most preferably 99% or more of the entire gelatin.

The gelatin may be natural gelatin or may be mutant gelatin of which at least one amino acid residue is different from that of natural gelatin. The natural gelatin means gelatin that is made from naturally occurring collagen or a polypeptide that contains the same amino acid sequence as gelatin made from naturally occurring collagen. Unless otherwise noted, in the present disclosure, mutant or recombinant gelatin will be collectively referred to as recombinant gelatin. Examples of the natural gelatin or the recombinant gelatin thereof include gelatin derived from animals, such as fish and mammals, and it is more preferable that the natural gelatin or the recombinant gelatin thereof is the natural gelatin of a mammal or the recombinant gelatin thereof. Examples of the mammal include a human, a horse, a pig, a mouse, a rat, and the like, and it is more preferable that the mammal is a human or a pig. It is preferable that the natural gelatin is gelatin derived from a pig or a human, and it is preferable that the recombinant gelatin is recombinant gelatin derived from a human.

Further, it is preferable that the gelatin is recombinant gelatin obtained in a case where a base sequence or an amino acid sequence, which is obtained from the modification of one or more bases or amino acid residues that is applied to a base sequence or an amino acid sequence of a gene encoding the collagen containing 6 or more consecutive sequences, each of which is represented by Gly-X-Y, is introduced into an appropriate host and expressed by an ordinary method. In a case where such recombinant gelatin is used, (bone) tissue repair ability is improved and various properties can be expressed as compared to a case where natural gelatin is used. For example, there is an advantage of avoiding an unfavorable influence, such as rejection from a living body.

For example, recombinant gelatin disclosed in EP1014176A, US6992172A, WO2004/85473A, WO2008/103041A, JP2010-519293A, JP2010-519252A, JP2010-518833A, JP2010-519251A, WO2010/128672A, WO2010/147109A, and the like can be particularly preferably used as the recombinant gelatin. Further, the recombinant gelatin has a molecular weight of preferably 2 kDa or more and 100 kDa or less, more preferably 5 kDa or more and 90 kDa or less, and still more preferably 10 kDa or more and 90 kDa or less.

In terms of biocompatibility, it is preferable that the recombinant gelatin further includes cell adhesion signals and it is more preferable that the recombinant gelatin includes two or more cell adhesion signals per molecule. Examples of such the cell adhesion signal can include the respective sequences, such as RGD sequences, LDV sequences, REDV sequences, YIGSR sequences, PDSGR sequences, RYVVLPR sequences, LGTIPG sequences, RNIAEIIKDI sequences, IKVAV sequences, LRE sequences, DGEA sequences, and HAV sequences; and preferably include RGD sequences, YIGSR sequences, PDSGR sequences, LGTIPG sequences, IKVAV sequences, and HAV sequences. It is particularly preferable that such the cell adhesion signal is the RGD sequences. It is more preferable that such the cell adhesion signal is ERGD sequence among the RGD sequences.

With regard to the arrangement of the RGD sequences in the recombinant gelatin, the number of amino acid residues between RGDs is preferably in a range of 0 to 100, and more preferably in a range of 25 to 60. Further, it is preferable that the RGD sequences are non-uniformly arranged within such a range of the number of amino acid residues. Furthermore, it is preferable that a ratio of the number of RGD sequences to the total number of amino acid residues in the recombinant gelatin is at least 0.4%. In a case where the recombinant gelatin contains 350 or more amino acid residues, it is preferable that each of stretches of 350 amino acid residues contains at least one RGD sequence.

The recombinant gelatin preferably contains at least two RGD sequences, more preferably includes at least three RGD sequences, and still more preferably includes at least four RGD sequences, per 250 amino acid residues. However, it is preferable that the sequences of the recombinant gelatin include the following aspects: (1) an aspect in which the sequences of the recombinant gelatin do not include a serine residue and a threonine residue, (2) an aspect in which the sequences of the recombinant gelatin do not include a serine residue, a threonine residue, an asparagine residue, a tyrosine residue, and a cysteine residue, and (3) an aspect in which the sequences of the recombinant gelatin do not include an amino acid sequence represented by Asp-Arg-Gly-Asp. The recombinant gelatin may include the preferable aspects (1) to (3) of sequences independently, or may include two or more of the aspects in combination. Further, the recombinant gelatin may be partially hydrolyzed.

It is preferable that the recombinant gelatin has a repeating structure of A-[(Gly-X-Y)n]m-B. m is in a range of 2 to 10, and is preferably in a range of 3 to 5. Each of A and B represents an arbitrary amino acid or amino acid sequence. n is in a range of 3 to 100, preferably in a range of 15 to 70, and more preferably in a range of 50 to 60.

Preferably, the recombinant gelatin is represented by Formula: Gly-Ala-Pro-[(Gly-X-Y)63]3-Gly (in the formula, 63 X's each independently represent any amino acid residue, and 63 Y's each independently represent any amino acid residue. Three (Gly-X-Y)63s may be the same or different from each other.).

It is preferable that a plurality of naturally occurring collagen sequence units are bonded to the repeating unit of the recombinant gelatin. Examples of naturally occurring collagen mentioned here preferably include type I, type II, type III, type IV, and type V. The naturally occurring collagen can be more preferably type I, type II, or type III. It is preferable that examples of the origin of the collagen can include a human, a horse, a pig, a mouse, a rat, and the like, and it is more preferable that the origin of the collagen is a human.

An isoelectric point of the recombinant gelatin can be preferably in a range of 5 to 10, more preferably in a range of 6 to 10, and still more preferably in a range of 7 to 9.5.

Preferred aspects of the recombinant gelatin can include the following aspects: (1) an aspect in which a carbamoyl group is not hydrolyzed, (2) an aspect in which procollagen is not included, (3) an aspect in which telopeptide is not included, and (4) a material for substantially pure collagen prepared using a nucleic acid encoding natural collagen. The recombinant gelatin may include the preferred aspects (1) to (4) independently, or may include two or more of the aspects in combination.

The recombinant gelatin can be preferably set to any one of the following (A) to (C) on the basis of the degree of (bone) tissue repair ability.

(A) Polypeptide that is represented by the following SEQ ID NO: 1,

GAP(GAPGLQGAPGLQGMPGERGAAGLPGPKGERGDAGPKGADGAPGAPGL
QGMPGERGAAGLPGPKGERGDAGPKGADGAPGKDGVRGLAGPIGPPGERGAAGLPG
PKGERGDAGPKGADGAPGKDGVRGLAGPIGPPGPAGAPGAPGLQGMPGERGAAGLP
GPKGERGDAGPKGADGAPGKDGVRGLAGPP)3G (SEQ ID NO: 1)

(B) Polypeptide that includes partial sequences having a sequence identity of 80% or more with a partial amino acid sequence consisting of 4th to 192nd amino acid residues in the amino acid sequence of (A) and has (bone) tissue repair ability, and

(C) Polypeptide that consists of an amino acid sequence in which one or several amino acid residues are deleted, substituted, or added to the amino acid sequence of (A) and has (bone) tissue repair ability.

The sequence identity of (B) can be more preferably 90% or more and still more preferably 95% or more from the viewpoint of the (bone) tissue repair ability of the recombinant gelatin.

The partial amino acid sequence of the sequence of (B) is a partial amino acid sequence corresponding to a repeating unit of the sequence represented by SEQ ID NO: 1. In a case where a plurality of partial amino acid sequences corresponding to the repeating unit are present in the polypeptide of (B), the polypeptide can include one, preferably two or more repeating units having a sequence identity of 80% or more.

Further, it is preferable that the polypeptide defined in (B) includes partial sequences having a sequence identity of 80% or more with the partial amino acid sequence corresponding to the repeating unit by 80% or more of the number of all amino acid residues as the total number of amino acid residues.

The length of the polypeptide defined in (B) can be the number of amino acid residues of 151 to 2260; is preferably the number of amino acid residues of 193 or more from the viewpoint of degradability after crosslinking and the number of amino acid residues of 944 or less from the viewpoint of stability; and is more preferably the number of amino acid residues of 380 to 756.

Further, the polypeptide defined in (C) may be a polypeptide that consists of an amino acid sequence in which one or several amino acid residues are deleted, substituted, or added to the amino acid sequence of (A) and has tissue repair ability.

One or several amino acid residues may be deleted, substituted, or added in the polypeptide defined in (C). Although varying depending on the total number of amino acid residues of the recombinant gelatin, the number of amino acid residues deleted, substituted, or added in the polypeptide defined in (C) can be, for example, in a range of 2 to 15 and preferably in a range of 2 to 5.

The recombinant gelatin can be manufactured by gene recombination techniques publicly known to those skilled in the art, and can be manufactured according to methods disclosed in, for example, EP1014176A, US6992172A, WO2004/85473A, WO2008/103041A, and the like. Specifically, a gene encoding an amino acid sequence of predetermined recombinant gelatin is acquired, this gene is incorporated into an expression vector to prepare a recombinant expression vector, and this recombinant expression vector is introduced into an appropriate host to prepare a transformant. The obtained transformant is cultured in an appropriate medium, so that recombinant gelatin is produced. Accordingly, in a case where the recombinant gelatin produced from a culture is recovered, the recombinant gelatin used in the present disclosure can be prepared.

-Polymer aqueous solution (liquid to be freeze-dried)-

The polymer aqueous solution (liquid to be freeze-dried) includes one or more types of polymers. The concentration of the polymers in the polymer aqueous solution is preferably 0.1% by mass or more, more preferably 1% by mass or more, and particularly preferably 5% by mass or more. In a case where the concentration of polymers in the polymer aqueous solution is lower than 0.1% by mass, it is difficult to maintain the structure of a polymer porous body after removing water. It is preferable that the polymer aqueous solution gels at a temperature equal to or higher than a freezing temperature. An upper limit of the concentration of the polymers in the polymer aqueous solution is not particularly limited as long as a polymer can be dissolved, and is generally 40% by mass or less or may be 30% by mass or less or 20% by mass or less.

In a case where a solution including polymers is purified and concentrated or dried polymers are dissolved in an aqueous medium, a polymer aqueous solution is prepared. (1) An polymer aqueous solution may be adjusted, or (2) a polymer aqueous solution adjusted in advance may be prepared and used. (3) A polymer aqueous solution obtained from purification and concentration may be freeze-dried, an aqueous medium may be added to an obtained freeze-dried body, and the freeze-dried body is redissolved to adjust the polymer aqueous solution. Alternatively, (4) a polymer aqueous solution obtained from purification and concentration may be frozen and an obtained frozen body may be thawed to adjust the polymer aqueous solution. It is preferable that the frozen body is thawed at a temperature of 30°C to 40°C for 15 to 20 hours from the viewpoint of reducing the generation of pores and insolubles (unthawed residues of the frozen body). The method of (4) is preferable from the viewpoint of reducing the time and effort required to adjust a polymer aqueous solution, the convenience of transport and storage, and reducing pores and insolubles in the polymer aqueous solution.

It is preferable that pores and insolubles dispersed in the polymer aqueous solution are removed before a freezing step by operations, such as filtration, centrifugation, decompression, and defoaming. Accordingly, a yield of a polymer aqueous solution-frozen body having low anisotropy is improved. The removal of pores and insolubles can be evaluated from the measurement of turbidity. Alternatively, the removal of pores and insolubles can also be evaluated from visual inspection using an optical microscope. For example, the number of pores and unnecessary substances appearing in the visual field of an optical microscope is calculated, and the removal of pores and insolubles can be evaluated from the number of pores and insolubles in 1 $\mu$L of the polymer aqueous solution. The number of pores or insolubles in the polymer aqueous solution is preferably 0.5 pieces/$\mu$L or less, more preferably 0.3 pieces/$\mu$L or less,

still more preferably 0.1 pieces/$\mu$L or less, and particularly preferably 0 piece/$\mu$L.

Components other than the polymers may be added to the polymer aqueous solution for the purpose of adding predetermined properties. Examples of such other components include components related to bone regeneration or new bone formation, such as a bone-inducing agent. Examples of the bone-inducing agent include a bone formation factor (BMP) and a basic fibroblast growth factor (bFGF), but the bone-inducing agent is not particularly limited. In addition, examples of the bone-inducing agent can include a crosslinking agent for a polypeptide or protein.

The aqueous medium of the polymer aqueous solution is not particularly limited as long as the aqueous medium can dissolve a polymer and can be used for biological tissue. Examples of the aqueous medium of the polymer aqueous solution can include those generally usable in the field of the art, such as water, saline, and a phosphate buffer solution.

In a case where gelatin is used as the polymer, the concentration of gelatin in a gelatin solution is not particularly limited as long as the gelatin can be dissolved. The concentration of the gelatin in the gelatin solution is, for example, preferably in a range of 0.5% by mass to 20% by mass, more preferably in a range of 2% by mass to 16% by mass, and still more preferably in a range of 4% by mass to 12% by mass. Further, the gelatin solution may be defoamed before the freezing step. Accordingly, it is possible to make ice crystals likely to be formed uniformly. A defoaming method is not particularly limited, and for example, vacuum centrifugal defoaming can be performed at a pressure of 2 to 10 kPa.

The gelatin solution may be filtered for the removal of undissolved particles. A filtration method is not particularly limited, and, for example, pressure filtration is performed using a filter having a pore diameter of 0.22 to 0.45 $\mu$m. A material of the filter is also not particularly limited, and polytetrafluoroethylene, polyethersulfone, cellulose acetate, polyvinylidene fluoride, or the like can be used. However, cellulose acetate is preferable from the viewpoint of low gelatin adsorbability and a small amount of eluate. A temperature at the time of preparing the gelatin solution is not particularly limited, and may be a commonly used temperature, for example, about 0°C to 60°C and preferably about 3°C to 40°C.

[Freeze-drying step]

A freeze-drying step is a combination of the following freezing step and a subsequent water removal step (drying step).

(Freezing step)

A step of freezing the polymer aqueous solution (liquid to be freeze-dried) stored in the freeze-drying container (hereinafter, referred to as a "freezing step") is performed. Accordingly, a polymer aqueous solution-frozen body can be obtained. Freezing means is not particularly limited, and the polymer aqueous solution may be frozen by a device, such as a refrigerator or a freeze dryer. In a case where the polymer aqueous solution is frozen by a freeze dryer, water can be continuously removed from the polymer aqueous solution-frozen body (the polymer aqueous solution-frozen body can be continuously freeze-dried) by the same device.

A temperature in the freezing step varies depending on the type of the polymer or the concentration of the polymer aqueous solution, and it is preferable that a temperature difference between a portion having the highest solution temperature and a portion having the lowest solution temperature in the liquid to be freeze-dried immediately before the generation of the heat of solidification (hereinafter, referred to as a "temperature difference immediately before the generation of the heat of solidification") is 2.5°C or less. Here, the "temperature difference immediately before the generation of the heat of solidification" means a maximum value of a temperature difference between the portion having the highest solution temperature and the portion having the lowest solution temperature between 1 second and 10 seconds before the generation of the heat of solidification. Further, the temperature of the portion having the lowest solution temperature in the liquid to be freeze-dried is preferably -5°C or less, more preferably -7.5°C or less, and still more preferably -10°C or less. Furthermore, it is preferable that the temperature of the portion having the lowest solution temperature is -15°C or more. It is most preferable that the temperature of the portion having the lowest solution temperature is in a range of -15°C to -7.5°C.

The temperature difference immediately before the generation of the heat of solidification can be measured as follows.

Thermocouple type thermometers are inserted at a plurality of positions in the liquid to be freeze-dried, and freeze-drying is performed while temperatures are recorded over time with a data logger. As a result, a temperature immediately before the generation of the heat of solidification can be measured. At that time, at least the temperature of a portion farthest from the first heat transfer body and the second heat transfer body and the temperature of a portion closest to the first heat transfer body and the second heat transfer body are measured, so that the "temperature difference immediately before the generation of the heat of solidification" can be measured.

Further, since the polymer aqueous solution-frozen body is manufactured as described above, probability of obtaining a polymer aqueous solution-frozen body having low anisotropy can be improved.

The "anisotropy" of the polymer aqueous solution-frozen body means a physical property measured as follows.

After the polymer aqueous solution-frozen body is freeze-dried, the vicinity of the center of a freeze-dried body (polymer porous body) is cut in a horizontal direction and a vertical direction. Then, each cross section is stained and a certain (2.0 mm × 2.0 mm or 2.5 mm × 2.5 mm) region is observed with an optical microscope. Among rectangles circumscribed in a region surrounded by a stained material in the observation region, a circumscribed rectangle in which a distance between two opposite sides is maximum is selected. 50 lengths of long sides of the circumscribed rectangles in which a distance between two opposite sides is maximum are measured in the observation region of each of the cross section taken in the horizontal direction and the cross section taken in the vertical direction, and an average of the measured lengths is defined as an average value of a long diameter of a mesh of the frozen body.

For each mesh at this time, a ratio d2/d1, which is obtained in a case where a smaller one of a long diameter (average value) in the cross section taken in the horizontal direction and a long diameter (average value) in the cross section taken in the vertical direction is denoted by d1 and the other thereof is denoted by d2, is defined as "anisotropy". In a case where this anisotropy is 3 or less, it is defined that "anisotropy is low".

From the viewpoint of manufacturing efficiency, it is preferable that a yield of a polymer aqueous solution-frozen body having low anisotropy is 90% or more.

(Water removal step (drying step))

A step of removing water from the polymer aqueous solution-frozen body (hereinafter, referred to as a "water removal step") is performed. Accordingly, a polymer porous body (freeze-dried body) can be obtained. Means for removing water is not particularly limited, examples of the means for removing water include a method of melting ice in the polymer aqueous solution-frozen body, a method of subliming the ice (freeze-drying the polymer aqueous solution-frozen body), and the like, and freeze-drying is preferable. The freeze-drying can be performed, for example, for 0.5 hours to 300 hours. There is no particular limitation on the freeze dryer that can be used.

Examples

The present disclosure will be more specifically described below using Examples. However, the present disclosure is not limited to these examples.

<Example 1>

[Freeze-drying container]

A freeze-drying container 100 shown in Figs. 1 and 2 was produced using an aluminum alloy (A5052) having external dimensions of 100 mm (length) × 106 mm (width) × 31 mm (height). The freeze-drying container 100 included a container body 10 (second heat transfer body, wall thickness: 5 mm, bottom thickness: 3 mm) that had internal dimensions of 90 mm (length) × 96 mm (width) × 28 mm (depth), and two plate-like first heat transfer bodies 30 (90 mm (length) × 3 mm (thickness) × 23 mm (height)) that were perpendicular to a bottom surface of the container body 10. Walls of the container body 10 and the first heat transfer bodies 30 were arranged at regular intervals (30 mm) in a lateral direction, the freeze-drying container 100 was provided with three storage portions 50 not communicating with each other, and the container body 10 was integrated with the first heat transfer bodies 30. Further, hydrophobic plating (a eutectic coating of a fluororesin and nickel, thickness: 10 μm) was performed on the entire freeze-drying container 100 with "NIFGRIP" (registered trademark) manufactured by ULVAC, Inc.

[Liquid to be freeze-dried]

A gelatin aqueous solution was prepared as liquid to be freeze-dried in the following manner.

The following recombinant peptide CBE3 (disclosed in WO2008/103041A) was prepared as the recombinant gelatin.

CBE3 molecular weight: 51.6 kD structure: GAP[(GXY)63]3G
Number of amino acids: 571 pieces
RGD sequence: 12 pieces
Imino acid content: 33%

Almost 100% of amino acids have a repeating structure of GXY. The amino acid sequence of CBE3 does not include a serine residue, a threonine residue, an asparagine residue, a tyrosine residue, and a cysteine residue. CBE3 includes an ERGD sequence.

A ratio of hydrophilic repeating units in a polymer having an isoelectric point of 9.34 is 26.1%.

Amino acid sequence (SEQ ID NO: 1)

A solution containing the recombinant gelatin was purified and then concentrated by ultrafiltration up to 4.0% by mass at a temperature of 30°C. After the obtained gelatin aqueous solution was freeze-dried, water for injection was added to a freeze-dried body, the temperature of the freeze-dried body was raised up to 37°C for 30 minutes, and the freeze-dried body was redissolved to obtain 7.5% by mass of a gelatin aqueous solution again. This gelatin aqueous solution was filtered using a cellulose acetate film filter of 0.22 μm, and was subjected to vacuum centrifugal defoaming for 180 seconds at a pressure of 4.0 kPa using a vacuum defoaming machine (Kurashiki Spinning Co., Ltd., KK-V300SS-I). The gelatin aqueous solution was sampled in a transparent container, which was made of polystyrene, to have a liquid thickness of 2.5 mm, and was observed with a visual field of 2.5 mm × 2.5 mm using an optical microscope from a lower surface of the liquid to an upper surface of the liquid in increments of 100 μm. As a result of observing 10 visual fields and calculating the average number of pores and insolubles, the number of pores was 0.42 pieces/μL and the number of insolubles was 0 piece/μL.

[Freeze-drying step]

20.5 g of the gelatin aqueous solution was poured into each of the three storage portions of the freeze-drying container. The volume of 20.5 g of the gelatin aqueous solution at a temperature of 23°C was 20.1 mL. The gelatin aqueous solution was stored in a measuring cylinder and a mass and a volume were compared with each other, so that the volume of 20.5 g of the gelatin aqueous solution at a temperature of 23°C was measured. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Example 2>

[Freeze-drying container]

A freeze-drying container 200 shown in Fig. 3 was produced using an aluminum alloy (A5052) having external dimensions of 100 mm (length) × 106 mm (width) × 31 mm (height). The freeze-drying container 200 included a container body 10 (second heat transfer body, wall thickness: 5 mm, bottom thickness: 3 mm) that had internal dimensions of 90 mm (length) × 96 mm (width) × 28 mm (depth), and four plate-like first heat transfer bodies 30 (90 mm (length) × 3 mm (thickness) × 23 mm (height)) that were perpendicular to a bottom surface of the container body 10. Walls of the container body 10 and the first heat transfer bodies 30 were arranged at regular intervals (16.8 mm) in a lateral direction, the freeze-drying container 100 was provided with five storage portions 50 not communicating with each other, and the container body 10 was integrated with the first heat transfer bodies 30. Further, hydrophobic plating (a eutectic coating of a fluororesin and nickel, thickness: 10 μm) was performed on the entire freeze-drying container 100 with "NIFGRIP" (registered trademark) manufactured by ULVAC, Inc.

[Liquid to be freeze-dried, freeze-drying step]

The same gelatin aqueous solution as the gelatin aqueous solution used in Example 1 was used, and 11.5 g of the gelatin aqueous solution was poured into each of the five storage portions of the freeze-drying container. The volume of 11.5 g of the gelatin aqueous solution at a temperature of 23°C was 11.3 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Example 3>

[Freeze-drying container]

A freeze-drying container was produced using an aluminum alloy (A5052) having external dimensions of 115 mm (length) × 115 mm (width) × 31 mm (height). The freeze-drying container included a container body (second heat transfer body, wall thickness: 5 mm, bottom thickness: 3 mm) that had internal dimensions of 105 mm (length) × 105 mm (width) × 28 mm (depth), and five plate-like first heat transfer bodies (105 mm (length) × 3 mm (thickness) × 25 mm (height)) that were perpendicular to a bottom surface of the container body. Walls of the container body and the first heat transfer bodies were arranged at regular intervals (15 mm) in a lateral direction, the freeze-drying container was provided with six storage portions not communicating with each other, and the container body was integrated with the first heat transfer bodies. Further, hydrophobic plating (a eutectic coating of a fluororesin and nickel, thickness: 10 μm) was performed on the entire freeze-drying container with "NIFGRIP" (registered trademark) manufactured by ULVAC, Inc.

[Liquid to be freeze-dried, freeze-drying step]

The same gelatin aqueous solution as the gelatin aqueous solution used in Example 1 was used, and 2.6 g of the gelatin aqueous solution was poured into each of the six storage portions of the freeze-drying container. The volume of 2.6 g of the gelatin aqueous solution at a temperature of 23°C was 2.55 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Example 4>

[Freeze-drying container]

A freeze-drying container was produced using an aluminum alloy (A5052) having external dimensions of 106 mm (length) × 106 mm (width) × 31 mm (height). The freeze-drying container included a container body (second heat transfer body, wall thickness: 5 mm, bottom thickness: 3 mm) that had internal dimensions of 96 mm (length) × 96 mm (width) × 28 mm (depth), and six plate-like first heat transfer bodies (96 mm (length) × 3 mm (thickness) × 25 mm (height)) that were perpendicular to a bottom surface of the container body. Walls of the container body and the first heat transfer bodies were arranged at regular intervals (11.1 mm) in a lateral direction, the freeze-drying container is provided with seven storage portions not communicating with each other, and the container body was integrated with the first heat transfer bodies. Further, hydrophobic coating (a eutectic coating of a fluororesin and nickel, thickness: 10 μm) was performed on the entire freeze-drying container with "NIFGRIP" (registered trademark) manufactured by ULVAC, Inc.

[Liquid to be freeze-dried, freeze-drying step]

The same gelatin aqueous solution as the gelatin aqueous solution used in Example 1 was used, and 7.6 g of the gelatin aqueous solution was poured into each of the six storage portions of the freeze-drying container. The volume of 7.6 g of the gelatin aqueous solution at a temperature of 23°C was 7.5 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Example 5>

[Freeze-drying container]

A freeze-drying container was produced using an aluminum alloy (A5052) having external dimensions of 106 mm (length) × 106 mm (width) × 31 mm (height). The freeze-drying container included a container body (second heat transfer body, wall thickness: 5 mm, bottom thickness: 3 mm) that had internal dimensions of 96 mm (length) × 96 mm (width) × 28 mm (depth), and seventh plate-like first heat transfer bodies (96 mm (length) × 3 mm (thickness) × 25 mm (height)) that were perpendicular to a bottom surface of the container body. Walls of the container body and the first heat transfer bodies were arranged at regular intervals (9.4 mm) in a lateral direction, the freeze-drying container was provided with eight storage portions not communicating with each other, and the container body was integrated with the first heat transfer bodies. Further, hydrophobic plating (a eutectic coating of a fluororesin and nickel, thickness: 10 μm) was performed on the entire freeze-drying container with "NIFGRIP" (registered trademark) manufactured by ULVAC, Inc.

[Liquid to be freeze-dried, freeze-drying step]

The same gelatin aqueous solution as the gelatin aqueous solution used in Example 1 was used, and 6.4 g of the gelatin aqueous solution was poured into each of the eight storage portions of the freeze-drying container. The volume of 6.4 g of the gelatin aqueous solution at a temperature of 23°C was 6.3 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Example 6>

[Freeze-drying container]

A freeze-drying container was produced in the same manner as in Example 5 except that hydrophobic coating was not performed with "NIFGRIP" (registered trademark), and the gelatin aqueous solution was freeze-dried to prepare freeze-dried bodies (polymer porous bodies).

<Example 7>

[Freeze-drying container]

A freeze-drying container was produced using an aluminum alloy (A5052) having external dimensions of 93 mm (length) × 93 mm (width) × 31 mm (height). The freeze-drying container included a container body (second heat transfer body, wall thickness: 5 mm, bottom thickness: 3 mm) that had internal dimensions of 83 mm (length) × 83 mm (width) × 28 mm (depth), and one plate-like first heat transfer body (83 mm (length) × 3 mm (thickness) × 25 mm (height)) that were perpendicular to a bottom surface of the container body. Walls of the container body and the first heat transfer body were arranged at regular intervals (40.0 mm) in a lateral direction, the freeze-drying container was provided with two storage portions not communicating with each other, and the container body was integrated with the first heat transfer body. Further, hydrophobic plating (a eutectic coating of a fluororesin and nickel, thickness: 10 $\mu$m) was performed on the entire freeze-drying container with "NIFGRIP" (registered trademark) manufactured by ULVAC, Inc.

[Liquid to be freeze-dried, freeze-drying step]

The same gelatin aqueous solution as the gelatin aqueous solution used in Example 1 was used, and 27.4 g of the gelatin aqueous solution was poured into each of the two storage portions of the freeze-drying container. The volume of 27.4 g of the gelatin aqueous solution at a temperature of 23°C was 26.9 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Comparative Example 1>

[Freeze-drying container]

A cylindrical cup-shaped freeze-drying container that was made of aluminum alloy (A5056), had an outer diameter of 106 mm, an inner diameter of 104 mm, a bottom thickness of 5 mm, and a height of 41.8 mm, and included an inner periphery of a bottom surface chamfered with R2 mm and an inner surface coated with FEP ("NF-004A" manufactured by Nippon Fusso Co., Ltd.) was prepared.

[Liquid to be freeze-dried, freeze-drying]

The same gelatin aqueous solution as the gelatin aqueous solution used in Example 1 was used, and 18.5 g of the gelatin aqueous solution was poured into the freeze-drying container. The volume of 18.5 g of the gelatin aqueous solution at a temperature of 23°C was 18.1 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of - 30°C, was vacuum-dried for 44 hours

EP 4 300 022 A1

at a temperature of -10°C, and was then vacuum-dried for 2 hours at a temperature of 20°C to remove water, so that freeze-dried bodies (polymer porous bodies) were prepared.

<Comparative Example 2>

[Freeze-drying container]

A freeze-drying container that included a container body having internal dimensions of 50 mm (length) $\times$ 50 mm (width) $\times$ 28 mm (depth) was produced using an aluminum alloy (A5052) having external dimensions of 60 mm (length) $\times$ 60 mm (width) $\times$ 31 mm (height). Further, hydrophobic coating (a eutectic coating of a fluororesin and nickel, thickness: 10 $\mu$m) was performed on the entire freeze-drying container 100 with "NIFGRIP" (registered trademark) manufactured by ULVAC, Inc.

[Liquid to be freeze-dried, freeze-drying]

The same gelatin aqueous solution as the gelatin aqueous solution used in Example 1 was used, and 40.1 g of the gelatin aqueous solution was poured into the freeze-drying container. The volume of 40.1 g of the gelatin aqueous solution at a temperature of 23°C was 39.3 mL, and was measured in the same manner as in Example 1. The freeze-drying container was put in a freeze dryer (DFR-5N-B manufactured by ULVAC, Inc.), was left for 2 hours at a temperature of 7°C, was cooled to -10°C, was vacuum-dried for 14 hours at a temperature of - 30°C, was vacuum-dried for 44 hours at a temperature of -10°C, and was then vacuum-dried for 2 hours at a temperature of 20°C. However, the gelatin aqueous solution was not completely dried and undried portions remained. For this reason, properties other than a drying property were not evaluated.

<Comparative Example 3>

[Freeze-drying container]

A freeze-drying container was produced using an aluminum alloy (A5052) having external dimensions of 97 mm (length) $\times$ 97 mm (width) $\times$ 31 mm (height). The freeze-drying container included a container body (second heat transfer body, wall thickness: 3 mm, bottom thickness: 3 mm) that had internal dimensions of 91 mm (length) $\times$ 91 mm (width) $\times$ 28 mm (depth), and one plate-like first heat transfer body (91 mm (length) $\times$ 3 mm (thickness) $\times$ 25 mm (height)) that was perpendicular to a bottom surface of the container body. Walls of the container body and the first heat transfer body were arranged at regular intervals (44.0 mm) in a lateral direction, the freeze-drying container was provided with two storage portions not communicating with each other, and the container body was integrated with the first heat transfer body.

[Liquid to be freeze-dried, freeze-drying step]

The same gelatin aqueous solution as the gelatin aqueous solution used in Example 1 was used, and 40.1 g of the gelatin aqueous solution was poured into each of the two storage portions of the freeze-drying container. The volume of 40.1 g of the gelatin aqueous solution at a temperature of 23°C was 39.3 mL, and was measured in the same manner as in Example 1. The freeze-drying container was put in a freeze dryer (DFR-5N-B manufactured by ULVAC, Inc.), was left for 2 hours at a temperature of 7°C, is cooled to -10°C, was vacuum-dried for 14 hours at a temperature of -30°C, was vacuum-dried for 44 hours at a temperature of -10°C, and was then vacuum-dried for 2 hours at a temperature of 20°C to remove water, so that freeze-dried bodies (polymer porous bodies) were prepared.

<Evaluation>

With regard to the freeze-dried bodies obtained as described above, a drying property, the uniformity of pores, and detachability were evaluated in the following manners.

[Drying property]

In the above-mentioned freeze-drying, a freeze-dried body having completely dried was determined as "P" (acceptable) and a freeze-dried body in which an undried portion remains was determined as "F" (unacceptable).

16

[Uniformity of pores]

The uniformity of pores was evaluated in the following manner.

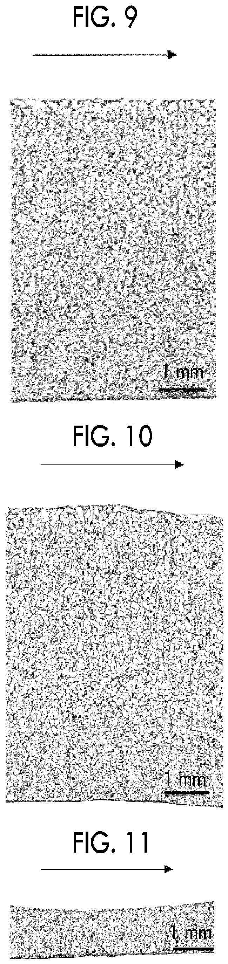

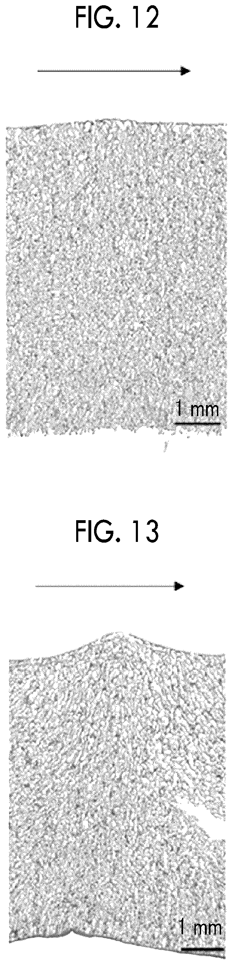

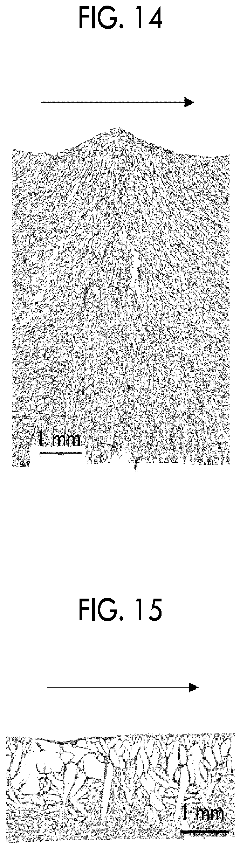

A specimen was produced for the observation of the pores of a freeze-dried body. Specifically, a freeze-dried body was cut into a size of 1 cm × 1 cm and is treated for 5 hours at a temperature of 135°C in a nitrogen atmosphere for the purpose of thermal crosslinking. Next, an obtained cross-linked body was immersed in saline for 12 hours, was embedded in an embedding agent for producing a frozen tissue section ("O.C.T. Compound" manufactured by Sakura Finetek Japan Co., Ltd.), and was then frozen at a temperature of -20°C. An obtained frozen block was sliced to a thickness of about 5 μm by a cryostat, so that a section was obtained. The section was attached to a slide glass, was dried for 5 minutes with cold air from a dryer, and was immersed in an eosin staining solution for 1 minute so that a cross section of the section was stained. The section was washed with water, was dehydrated by being immersed in ethanol, was cleared by being immersed in xylene, was sealed with marinol and a cover glass, and was then dried for 2 hours at room temperature, so that a specimen was obtained. A region having a size of 2.5 mm × 2.5 mm on the specimen was observed with an optical microscope and optical microscope images shown in Figs. 9 to 15 were obtained. In the optical microscope images shown in Figs. 9 to 15, a direction of an arrow indicates an in-plane direction of a liquid surface of the liquid to be freeze-dried that is a precursor of the freeze-dried body.

Evaluation criteria are as follows. Practical levels are S and P.

-Evaluation criteria-

S: Pores are uniform and there is no bulge in the direction of the liquid surface.

P: Pores are substantially uniform but a small bulge is identified in the direction of the liquid surface.

F: Pores are non-uniform.

[Detachability]

The detachability of a freeze-dried body from the freeze-drying container was evaluated on the basis of the state of the freeze-dried body when the freeze-dried body was taken out of the freeze-drying container. Both S determination and P determination are acceptable.

Evaluation criteria are as follows. Practical levels are S and P.

-Evaluation criteria-

S: A crack does not occur in the freeze-dried body.

P: A crack occurs in the freeze-dried body but the freeze-dried body is not ruptured.

F: The freeze-dried body is ruptured.

The evaluation results of a drying property, the uniformity of pores, and detachability are shown in Table 1.

[Table 1]

| | Example 1 | Example 2 | Example 3 | Example 4 | Example 5 | Example 6 | Example 7 | Comparative example 1 | Comparative example 2 | Comparative example 3 |
|---|---|---|---|---|---|---|---|---|---|---|
| Shortest distance (mm) | 15 | 8.4 | 7.5 | 5.6 | 4.7 | 4.7 | 20 | - | - | 22 |
| Ratio of liquid contact area to liquid volume ($mm^2/mL$) | 223 | 275 | 770 | 343 | 377 | 377 | 198 | - | - | 169 |
| Uniformity of pores | S | S | S | S | P | P | P | F | - | F |
| Drying property | P | P | P | P | P | P | P | P | F | P |
| Hydrophobic film | NIFGRIP | NIFGRIP | NIFGRIP | NIFGRIP | NIFGRIP | NONE | NONE | FEP | NIFGRIP | - |
| Detachability | S | S | P | S | S | P | P | S | - | - |

In a case where the freeze-drying containers of Examples 1 to 7 were used, freeze-dried bodies including uniform pores could be obtained as shown in Table 1.

The first shortest distance and the second shortest distance were 5 mm or more in Examples 1 to 4. Further, the ratio of a liquid contact area to a liquid volume was 200 mm$^2$/mL or more in Examples 1 to 4. For this reason, Examples 1 to 4 were more excellent in the uniformity of pores.

It is found that Examples 1 to 4 in which the first shortest distance and the second shortest distance are 5 mm or more are more excellent in the uniformity in pores than Examples 5 and 6 in which the first shortest distance and the second shortest distance are less than 5 mm.

Further, it is found that Examples 1 to 4 in which the ratio of a liquid contact area to a liquid volume is 200 mm$^2$/mL or more are more excellent in the uniformity in pores than Example 7 in which the ratio of a liquid contact area to a liquid volume is less than 200 mm$^2$/mL.

Since the ratio of a liquid contact area to a liquid volume in each of Examples 1, 2, 4, and 5 was 500 mm$^2$/mL or less, Examples 1, 2, 4, and 5 were more excellent in detachability than Example 3.

It was found from the comparison between Examples 5 and 6 that Example 5 including a hydrophobic coating was more excellent in detachability.

On the other hand, since a heat transfer body other than the container body was not provided in Comparative Example 1, pores were non-uniform.

Since a heat transfer body other than the container body was not provided and the amount of gelatin aqueous solution was large in Comparative Example 2, the gelatin aqueous solution could not be completely dried and undried portions remained.

Since points at which both the first shortest distance and the second shortest distance from an arbitrary point on the liquid surface of the liquid to be freeze-dried exceed 20 mm were included in Comparative Example 3, pores were non-uniform.

The entire content of the present disclosure of Japanese Patent Application No. 2021-054136, filed March 26, 2021, is incorporated in this specification by reference. All documents, patent applications, and technical standards disclosed in this specification are incorporated in this specification by reference such that the incorporation of each of the documents, the patent applications, and the technical standards by reference is specific and is as detailed as each of the documents, the patent applications, and the technical standards. [Sequence listing] International application 21F00245W1JP22013715_1.app based on International Patent Cooperation Treaty

**Claims**

1. A freeze-drying container comprising:

   a container body that stores liquid to be freeze-dried; and
   a first heat transfer body that is in contact with the liquid to be freeze-dried and a cold source,
   wherein the container body is a non-heat transfer body or a second heat transfer body of which at least a part is in contact with a liquid surface of the liquid to be freeze-dried and the cold source, and
   at least one of a first shortest distance between an arbitrary point on the liquid surface of the liquid to be freeze-dried and the first heat transfer body or a second shortest distance in the liquid surface between the arbitrary point and the second heat transfer body is 20 mm or less.

2. The freeze-drying container according to claim 1,
   wherein a ratio of an area of portions of the first heat transfer body and the second heat transfer body, which are in contact with the liquid to be freeze-dried, to a volume of the liquid to be freeze-dried stored in the freeze-drying container at a temperature of 23°C is 200 mm$^2$/mL or more.

3. The freeze-drying container according to claim 1 or 2,
   wherein a thermal conductivity of the first heat transfer body at a temperature of 0°C is 15 W/m•K or more.

4. The freeze-drying container according to any one of claims 1 to 3,
   wherein a thermal conductivity of the second heat transfer body at a temperature of 0°C is 15 W/m•K or more.

5. The freeze-drying container according to any one of claims 1 to 4,
   wherein the first heat transfer body is disposed in a direction perpendicular to a bottom surface of the container body.

6. The freeze-drying container according to any one of claims 1 to 5,

wherein at least a part of portions of the container body and the first heat transfer body, which are in contact with the liquid to be freeze-dried, include a hydrophobic film.

7. The freeze-drying container according to claim 6,
   wherein an entirety of the container body and the first heat transfer body includes the hydrophobic film.

8. The freeze-drying container according to claim 6 or 7,
   wherein a thickness of the hydrophobic film is 1 mm or less.

# FIG. 1

# FIG. 2

100

30    30

A                    A

10

50    50    50

# FIG. 3

200

52

30    52    30

52

52

10

52  30    52  30

## FIG. 4

300

32

10

## FIG. 5

300

32

10

# FIG. 6

# FIG. 7

# FIG. 8

## FIG. 9

1 mm

## FIG. 10

1 mm

## FIG. 11

1 mm

## FIG. 12

## FIG. 13

FIG. 14

FIG. 15

## INTERNATIONAL SEARCH REPORT

| International application No. |
| --- |
| **PCT/JP2022/013715** |

**A. CLASSIFICATION OF SUBJECT MATTER**

*F26B 25/06*(2006.01)i; *A23L 3/44*(2006.01)i; *C07K 14/47*(2006.01)i; *C12M 1/00*(2006.01)i; *F26B 5/06*(2006.01)i
FI: F26B25/06 Z; F26B5/06; C07K14/47; A23L3/44 ZNA; C12M1/00 Z

According to International Patent Classification (IPC) or to both national classification and IPC

**B. FIELDS SEARCHED**

Minimum documentation searched (classification system followed by classification symbols)

F26B25/06; A23L3/44; C07K14/47; C12M1/00; F26B5/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | US 2018/0050109 A1 (TUFTS UNIVERSITY) 22 February 2018 (2018-02-22) paragraphs [0294]-[0298], fig. 5, 6 | 1-5 |
| Y | | 6-8 |
| X | JP 8-327229 A (MUTUAL CORP.) 13 December 1996 (1996-12-13) paragraph [0007], fig. 4 | 1-5 |
| Y | | 6-8 |
| Y | US 2002/0081408 A1 (SPALLER, Michael) 27 June 2002 (2002-06-27) paragraph [0034] | 6-8 |
| X | JP 2-306088 A (FUJITSU LTD.) 19 December 1990 (1990-12-19) page 2, lower right column, lines 11-17, fig. 1, 2 | 1-5 |
| Y | | 6-8 |
| X | US 4603492 A (HAG GF AKTIENGESELLSCHAFT) 05 August 1986 (1986-08-05) column 3, line 32 to column 4, line 41, fig. 1-4 | 1-5 |
| Y | | 6-8 |

☑ Further documents are listed in the continuation of Box C.      ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **02 June 2022** | **14 June 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/013715** |

## C.    DOCUMENTS CONSIDERED TO BE RELEVANT

| Category* | Citation of document, with indication, where appropriate, of the relevant passages | Relevant to claim No. |
| --- | --- | --- |
| A | JP 47-9666 B1 (LEYBOLD HERAEUS GMBH) 22 March 1972 (1972-03-22)<br>entire text, all drawings | 1-8 |
| A | CN 211625874 U (WUHAN METWARE MEDICAL TECHNOLOGY CO., LTD.) 02 October 2020 (2020-10-02)<br>entire text, all drawings | 1-8 |
| A | CN 201548033 U (LIAO, Hanxiong) 11 August 2010 (2010-08-11)<br>entire text, all drawings | 1-8 |
| A | CN 210921979 U (GUANGZHOU LINGXIAN BIO-TECH CO., LTD.) 03 July 2020 (2020-07-03)<br>entire text, all drawings | 1-8 |

Form PCT/ISA/210 (second sheet) (January 2015)

| **INTERNATIONAL SEARCH REPORT** | International application No. |
|---|---|
| | **PCT/JP2022/013715** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.  ☑ forming part of the international application as filed:

        ☑  in the form of an Annex C/ST.25 text file.

        ☐  on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2. ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

# EP 4 300 022 A1

<table>
<tr><td colspan="2">INTERNATIONAL SEARCH REPORT<br>Information on patent family members</td><td colspan="2">International application No.<br><br>**PCT/JP2022/013715**</td></tr>
</table>

| Patent document cited in search report | Publication date (day/month/year) | Patent family member(s) | Publication date (day/month/year) |
|---|---|---|---|
| US 2018/0050109 A1 | 22 February 2018 | US 2021/0177977 A1<br>WO 2016/145281 A1<br>CA 2979507 A1<br>KR 10-2017-0126470 A | |
| JP 8-327229 A | 13 December 1996 | (Family: none) | |
| US 2002/0081408 A1 | 27 June 2002 | EP 949150 A2<br>DE 19815993 A1 | |
| JP 2-306088 A | 19 December 1990 | (Family: none) | |
| US 4603492 A | 05 August 1986 | EP 158322 A2<br>DE 3413856 A1 | |
| JP 47-9666 B1 | 22 March 1972 | (Family: none) | |
| CN 211625874 U | 02 October 2020 | (Family: none) | |
| CN 201548033 U | 11 August 2010 | (Family: none) | |
| CN 210921979 U | 03 July 2020 | (Family: none) | |

Form PCT/ISA/210 (patent family annex) (January 2015)

31

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62101728 U
- JP S62101728 U
- EP 1014176 A
- US 6992172 A
- WO 200485473 A
- WO 2008103041 A
- JP 2010519293 A
- JP 2010519252 A
- JP 2010518833 A
- JP 2010519251 A
- WO 2010128672 A
- WO 2010147109 A
- JP 2021054136 A
- JP 22013715 B